# EUROPEAN PATENT SPECIFICATION

(11) **EP 2 831 781 B1**
(45) Date of publication and mention of the grant of the patent: **30.12.2020**
(21) Application number: 13723958.8
(22) Date of filing: 22.03.2013
(51) Int. Cl.: G16H 10/20, G16H 10/60

(54) **METHOD FOR SYNCHRONIZING THE STATE OF A COMPUTER INTERPRETABLE GUIDELINE ENGINE WITH THE STATE OF PATIENT CARE**
VERFAHREN ZUR SYNCHRONISIERUNG DES ZUSTANDES EINER MASCHINE MIT COMPUTERINTERPRETIERBAREN RICHTLINIEN MIT DEM PATIENTENPFLEGEZUSTAND
PROCÉDÉ DE SYNCHRONISATION DE L'ÉTAT D'UN MOTEUR DE LIGNE DE GUIDAGE POUVANT ÊTRE INTERPRÉTÉ PAR ORDINATEUR AVEC L'ÉTAT DE SOINS DE PATIENT

(30) Priority: 30.03.2012 US 201261617718 P
(43) Date of publication of application: 04.02.2015
(73) Proprietor: Koninklijke Philips N.V., 5656 AG Eindhoven (NL)
(72) Inventor: VAN ZON, Cornelis Conradus Adrianus Maria, NL-5656 AE Eindhoven (NL); DUTTA, Pradyumna, NL-5656 AE Eindhoven (NL)
(74) Representative: Philips Intellectual Property & Standards
(86) International application number: PCT/IB2013/052284
(87) International publication number: WO 2013/144796

(56) References cited:
- WO-A2-02/19247
- WO-A2-02/37337
- WO-A2-03/040990
- WO-A2-2011/070462

## Description

The present application relates to clinical decision making. It finds particular application in conjunction with clinical decision support systems and will be described with particular reference thereto. However, it is to be understood that it also finds application in other usage scenarios and is not necessarily limited to the aforementioned application.

Clinical protocols (also referred to as care protocols) are the procedures established by medical institutions, such as hospitals, to follow when caring for patients. These protocols aim at ensuring the best possible care for patients while minimizing the cost of care. Typically, clinical protocols are derived from clinical guidelines (GLs). Clinical guidelines are recommendations on the appropriate treatment and care for people with specific diseases and conditions based on the best available evidence. The clinical guidelines consist of recommendations and decision criteria for diagnosis, management, and treatment of patients suffering from these diseases and conditions. Modern guidelines represent evidence-based medicine, i.e., they are based on clinical evidence acquired through scientific methods and studies such as randomized clinical trials. Evidence-based practice is the foundation of modern guidelines and their application.

Studies have shown that adhering to the recommendations of clinical guidelines reduces costs and improves outcomes. As a result, guideline adherence is increasingly tied to performance measures and reimbursements. This creates an opportunity for healthcare solutions, in particular clinical decision support (CDS) systems, to support this trend. For such solutions to be successful they have to fit seamlessly into existing clinical workflow. Such solutions can be achieved by representing clinical guidelines, and local care protocols that are derived from them, in a formal way so that they can be interpreted by computers.

The resulting computer interpretable guidelines (CIGs), also known as executable clinical guidelines, are computer interpretable representations of the clinical knowledge contained in guidelines. A CIG is typically comprised of a plurality of care steps and incorporates recommendations as to how to treat and/or care for a patient based on the present state of the patient as represented by patient data. Further, a CIG typically embodies a clinical protocol of the medical institution employing the CIG. Therefore, CIGs embodying clinical protocols derived from clinical guidelines support medical institutions in adhering to the clinical guidelines. Software that can execute a CIG is called a CIG engine or a guideline execution engine, and is typically part of (or, if provided as an external service: invoked by) a CDS application. This engine applies a CIG's logic to patient data and user input in order to generate recommendations for care providers. The generated recommendations including alerts, notifications, messages, reminders, suggestions, and the like.

When a CIG-based CDS tool is used for patient care, and the user starts a new patient case on the tool, any new patient data that becomes available will be sent to the tool's CIG engine. Such data may be of clinical nature (e.g., patient history, vitals) or of workflow nature (e.g., MRI scan evaluated by radiologist, blood samples taken and sent to lab) and may originate from within the CDS tool (e.g., entered by the user) or from outside of it (e.g., entered into an enterprise electronic medical record and communicated to the CDS tool). Upon receiving new patient data, the CIG engine applies that data to the CIG's logic, updates its status accordingly, and provides any resulting recommendations to the CDS tool, which signals them to the user and/or dispatches them to its environment (e.g., to an order entry system).

WO 02/37337 deals with a system and method for automatically integrating data with staged diabetes management guidelines to generate displays containing guidelines and data. Different kind of data, such as patient data and statistical data, are employed from different locations and integrated in a database along with guidelines. Missing data may be inferred inter alia from users or from other sources.

WO 03/040990 pertains to a method and system including a data source containing patient records, a guideline knowledge base containing clinical guidelines and a quality adherence engine for monitoring adherence with the clinical guidelines for the patients being treated. Patient records may include information derived from mining unstructured patient data.

In some cases, the CIG-based CDS tool does not get deployed immediately when a patient enters the hospital. For example, the tool may not be available because a workstation crashed, the number of licenses was exceeded, no trained user was available, care providers were too busy to use the tool, the tool was not applicable because it is specific to a diagnosis that had not yet been made, the tool gets used for a patient who had previously been treated elsewhere before coming to the current healthcare facility, the tool is introduced to the healthcare facility for the first time and must create CIG instances for all existing patients under treatment, and the like. In such scenarios the tool gets invoked partway through patient care, in which case the initially 'blank' CIG state may not properly reflect the patient's actual state of care, which can lead to undesired behavior of the CIG engine and medical errors. To prevent this from happening, the CIG engine must be put in the appropriate state when invoked for a given patient. Presently, to accomplish this, the user explicitly indicates the proper entry point into the guideline.

The present application provides new and improved methods and systems which overcome the above-referenced problems and others by inferring the CIG entry point from existing patient data.

The invention is defined by the independent claims. Advantageous embodiments are described in the dependent claims. While several embodiments and/or examples are disclosed in this description, the subject matter for which protection is sought is strictly and solely limited to those embodiments and/ or examples encompassed by the scope of the appended claims. Embodiments and/or examples mentioned in the description that do not fall under the scope of the claims are useful for understanding the invention.

In accordance with one aspect, a method of synchronizing a state of a computer interpretable guideline engine with a state of patient care is provided. The method including receiving historic patient data, including workflow data and clinical data, for one or more patients, wherein the workflow data includes information on a plurality of care steps for each of the patients and the clinical data includes clinical measurements and observations collected from each of the patients, and synchronizing the state of the computer interpretable guideline engine by processing the workflow and clinical data in chronological order, for each of the patients.

In accordance with another aspect, a system for managing clinical protocols and/or clinical guidelines is provided. A data collection engine receives patient data, including current workflow data and clinical data for a plurality of patients, and can request historic workflow data and clinical data from a patient information system. The workflow data includes information on a plurality of care steps for each of the patients and the clinical data includes clinical measurements and observations collected from each of the patients. The patient information system stores historic workflow data and clinical data for the plurality of patients. A guideline execution engine processes new patient data to generate recommendations for care providers. In case it is first invoked when historic patient data exists, i.e., part-way patient care, it can use the available historic patient data to synchronize itself with the current state of patient care.

In accordance with another aspect, a clinical decision support or workflow management system is provided. A data collection engine collects current workflow data and clinical data for at least one patient. The workflow data includes information on at least a current care step of a sequence of care steps and the clinical data includes clinical measurements and observations collected from the patient over time. A patient information system stores historic workflow data and clinical data for the plurality of patients. The logic to make recommendations to care providers about subsequent care steps is based on clinical guidelines and/or protocols and is formalized in one or more computer interpretable guidelines; the recommendations are made by a guideline execution engine applying available workflow data and clinical data to aforementioned logic. In case the guideline execution engine is first invoked when historic patient data exists, it can use the available historic patient data to synchronize itself with the current state of patient care.

Missing data is ranked by Principal Components Analysis to determine relevant questions to ask the users. A priori segmentation of the computer interpretable guideline is performed to determine which patient data can be inferred and which patient data needs to be provided by the users.

One advantage of synchronizing the state of a computer interpretable guideline engine with the state of patient care resides in providing up-to-date recommendations.

Another advantage resides in providing retroactive recommendations.

Another advantage resides in providing optimum use of computer interpretable guidelines at any state of patient care.

Another advantage resides in improved patient care.

The invention may take form in various components and arrangements of components, and in various steps and arrangements of steps. The drawings are only for purposes of illustrating the preferred embodiments and are not to be construed as limiting the invention.
FIGURE 1 is a block diagram of an information technology (IT) infrastructure of a medical institution according to aspects of the present disclosure;
FIGURE 2 is a block diagram of functional components of a clinical decision support and/or workflow management (CDS/WM) system according to aspects of the present disclosure;
FIGURE 3 is a block diagram of structural components of a CDS/WM system according to aspects of the present disclosure;
FIGURE 4 illustrates the synchronization procedure of a CDS/WM system according to aspects of the present disclosure.

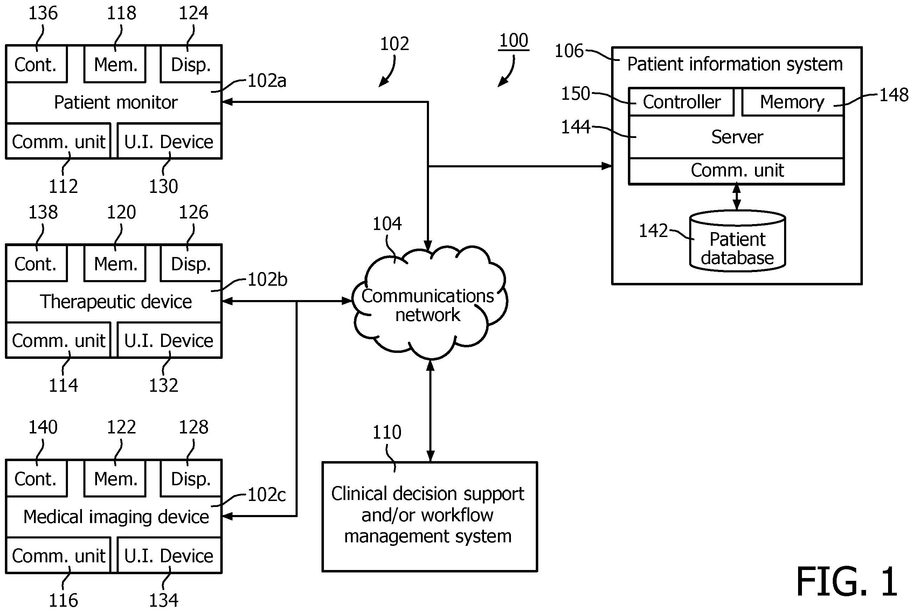

With reference to FIGURE 1, a block diagram of an information technology (IT) infrastructure **100** of a medical institution, such as a hospital, is provided. The IT infrastructure **100** typically includes one or more clinical devices **102,** a communications network **104,** a patient information system **106,** a clinical decision support and/or workflow management (CDS/WM) system **110,** and the like. However, it is to be understood that more or less components and/or different arrangements of components are contemplated.

The clinical device(s) **102** include one or more of patient monitors, devices at patient beds, mobile communications devices carried by clinicians, clinician workstations, settings of treatment providing equipment, and the like at various physical locations in the medical institution. Further, each of the clinical device(s) **102** is associated with one or more patients and/or one or more clinicians. For example, a patient monitor attached to a patient and/or a clinician's workstation configured to receive clinical knowledge for a plurality of patients. Each of the patient(s) associated with the clinical device(s) **102** is associated with one or more clinical problems, such as diseases or medical conditions.

As illustrated, the clinical device(s) **102** include a patient monitor **102a,** a therapeutic device **102b,** and a medical imaging device **102c.** Others, of course, are contemplated. Communications units **112, 114, 116** of the clinical device(s) **102** facilitate communication with external systems and/or databases, such as the CDS/WM system **110,** via the communications network **104.** Memories **118, 120,122** of the clinical device(s) **102** store executable instructions for performing one of more of the functions associated with the clinical device(s) **102.** Displays **124, 126, 128** of the clinical device(s) **102** allow the clinical device(s) **102** to display data and/or messages for the benefit of corresponding users. User input devices **130, 132, 134** of the clinical device(s) **102** allow the corresponding users of the clinical device(s) **102** to interact with the clinical device(s) **102** and/or respond to messages displayed on the displays **124, 126, 128.** Controllers **136, 138, 140** of the clinical device(s) **102** execute instructions stored on the memories **118, 120, 122** to carry out the functions associated with the clinical device(s) **102.**

The communications network **104** allows communication between components of the medical institution connected thereto, such as the CDS/WM system **110** and the clinical device(s) **102,** and is suitable for the transfer of digital data between the components. Suitably, the communications network **104** is a local area network. However, it is contemplated that the communications network **104** is one or more of the Internet, a wide area network, a wireless network, a wired network, a cellular network, a data bus, such as USB and I2C, and the like.

The patient information system **106** acts as a central repository of electronic medical records (EMRs) for patient data. Patient data from the clinical device(s) **102** and other devices generating patient data are suitably stored in the patient information system **106.** In some instances, patient data are received directly from the source of the patient data, and, in other instances, patient data are received indirectly from the source of the patient data. For example, patient data generated by one of the clinical device(s) **102** are received indirectly from the CDS/WM system **110.**

Typically, the patient information system **106** includes one of more of a database **142,** a server **144,** and the like. The database **142** stores EMRs for patients of the medical institution. The server **144** allows components of the medical institution to access the EMRs via the communications network **104.** A communications unit of the server **144** facilitates communication between the server **144** and external devices, such as the clinical device(s) **102,** via the communications network **104.** The communications unit **146** further facilitates communication with the database **142** of the patient information system **106.** A memory **148** of the server **144** stores executable instructions for performing one of more of the functions associated with the server **144.** A controller **150** of the server **144** executes instructions stored on the memory **148** to carry out the functions associated with the server **144.**

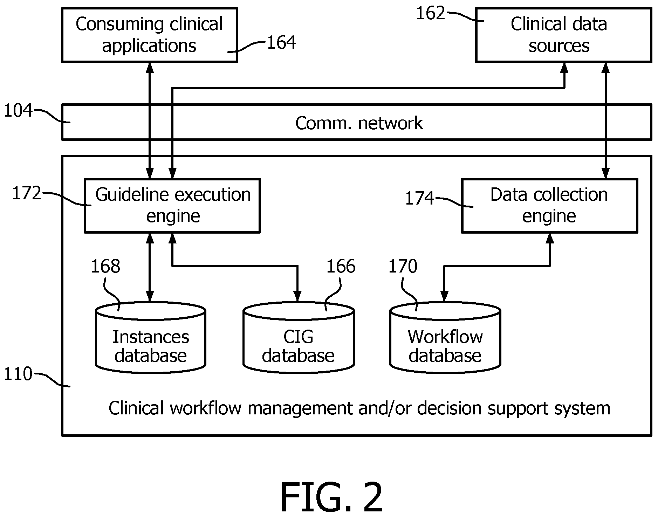

The CDS/WM system **110** receives patient data from one or more clinical data sources **162** (see Fig. 2) and, in certain embodiments, provides clinical recommendations based on clinical protocols and/or clinical guidelines to one or more consuming clinical applications **164** (see Fig. 2) to promote adherence to the clinical protocols and/or clinical guidelines. It is also contemplated that the CDS/WM system **110** be a shared service or part of and local to a specific clinical device.

The clinical data sources **162** provide patient data for associated patients to the CDS/WM system **110.** The patient data suitably includes clinical data, such as patient symptoms (e.g., chief complaints), patient findings (e.g., physical exam findings), laboratory data (e.g., creatinine values), physiological data (e.g., blood pressure), workflow data, identification data (e.g., patient IDs), and the like. The patient data (both clinical data and workflow data) is documented electronically with time stamps and is accessible by the CDS/WM system **110.** It is contemplated that the workflow data identifies, for example, one or more of care steps performed, care steps currently being performed, care steps yet to be performed, and the like. Suitably, clinical data sources **162** provide patient data to the CDS/WM system **110** as it becomes available. For example, if a patient monitor collects data from associated sensors every 5 seconds, newly collected patient data is provided every 5 seconds. However, it is contemplated that events other than the availability of patient data, such as timer events, workflow events, user input events, and the like result in the provisioning of patient data.

The consuming clinical applications **164** receive clinical recommendations for associated patients from the CDS/WM system **110.** The clinical recommendations may include reminders, alerts, suggested next steps, background information, drug choices and dosages, and the like, that aim to assist clinicians with the treatment of the associated patients. To receive clinical recommendations for a patient, a consuming clinical application suitably registers with the CDS/WM system **110** to receive clinical recommendations for the patient.

The clinical data sources **162** suitably include at least one of: (1) one or more of the clinical devices **102;** (2) the patient information system **106;** (3) one or more of the auxiliary systems **108;** (4) other devices and/or applications generating patient data; (5) the CDS/WM system **110,** such as a user input device thereof; and (6) the like. The consuming clinical applications **164** suitably include at least one of: (1) one or more of the clinical devices **102;** (2) the patient information system **106;** (3) one or more of the auxiliary systems **108;** (4) applications running on devices (e.g., PCs, cell-phones, etc.); (5) the CDS/WM system **110;** and (6) the like. In certain embodiments, one or more of the components of the IT infrastructure **100** belong to both the clinical data sources **162** and the consuming clinical applications **164.** It is also contemplated that the clinical devices **102** are both a producer and a consumer of patient data.

The CDS/WM system **110,** as discussed in detail below, utilizes an algorithm or procedure for synchronizing the state of a CIG engine with the state of patient care. Specifically, the CDS/WM system **110** applies CIG logic to historic patient data in chronological order and properly handles timer events and missing data. Specifically, in the case, the guideline execution engine **172** is involved partway through patient care, in which case an initially 'blank' CIG state may not properly reflect the patient's actual state of care, the guideline execution engine **172** executes the CIG on historic patient data in chronological order. It is also contemplated that CDS/WM system **110** is any CIG-based CDS system that has access to or can be provided with historic patient data. More generally, it is also contemplated that the CDS/WM system **110** is used by protocol engines with access to historic data.

It is contemplated that the CDS/WM system **110** includes one or more devices, servers, computers, database, and the like implementing varying functional aspects of the CDS/WM system **110,** described in detail hereafter. Further, although described as part of the CDS/WM system **110,** it is contemplated that the tracking/managing and review of a patient case is performed by a component other than the CDS/WM system **110.**

With reference to FIGURE 2, a detailed view of the functional components of the CDS/WM system **110** according to aspects of the present disclosure is provided. The CDS/WM system **110** suitably includes a computer interpretable guideline (CIG) database **166,** a CIG instances database **168,** a workflow database **170,** a guideline execution engine **172,** a data collection engine **174,** and the like. It is to be appreciated these functional components are merely abstractions to simplify the discussion hereafter and are not intended to be construed as limiting the structural layout of the CDS/WM system **110.**

The guideline execution engine **172** executes CIGs embodying clinical protocols of the medical institution. A clinical protocol typically includes one or more preferred care steps and logic to decide on the timing or sequence for an occurrence of the care step(s) as a function of patient information and clinical problem. Further, a clinical protocol typically includes recommendations to perform specific care steps, with associated instructions. It is contemplated that the clinical protocols are derived from clinical guidelines, but other approaches to deriving the clinical protocols are contemplated. Suitably, the CIGs are stored within the CIG database **166** and indexed by clinical problem. However, it is contemplated that the CIGs are stored in other components of the medical institution.

To execute CIGs, the guideline execution engine **172** creates instances of the CIGs stored in the CIG database **166** relevant to the clinical problems associated with the patients serviced by the medical institution. For example, when a patient with a particular clinical problem is admitted to the medical institution, the CDS/WM system **110** locates one or more ones of the CIGs in the CIG database **166** relevant to the patient and creates an instance for each one of more of these CIG for the patient. CIG selection can either be done automatically by the CDS/WM system or a CDS application, or manually by the care provider. An instance of a CIG is the CIG-specific state of the guideline execution engine tailored to a particular patient by applying the available electronic clinical and workflow data for that patient to the CIG's logic. The instances are suitably maintained in the instances database **168** and indexed by patient. However, it is contemplated that the instances are stored in other components of the medical institution.

The guideline execution engine **172** further maintains and/or updates the instances of the CIGs. As patient data relevant to one or more of the instances becomes available, the one or more instances are updated to reflect the updated patient information. For example, as a care step is performed for a particular patient, it is contemplated that one or more associated instances are updated to reflect that the care step has been performed. Relevant patient data includes one or more of clinical data, workflow data, and the like. It is contemplated that the patient data is received directly from components of the medical institution, such as the sourcing clinical device(s), or indirectly via a component of the medical institution, such as the patient information system **106.**

While the guideline execution engine **172** is executing the CIGs, the guideline execution engine **172** provides clinical knowledge based on the CIGs to the consuming medical device(s) and/or other components of the medical institution. It is also contemplated that the CDS/WM system **110** itself may be the only consuming medical device and provides recommendations and instructions to the user through its display. As noted above, a CIG typically includes recommendations for care steps. Hence, as an instance of a CIG is updated by, for example, processing the completion of a care step, recommendations and/or instructions for subsequent care steps are provided to relevant one or more of the consuming medical device(s). In certain embodiments, the relevant consuming medical device(s) are the consuming medical device(s) that registered with the CDS/WM system **110** to receive clinical knowledge pertaining to a patient.

The data collection engine **174** collects workflow data pertaining to workflows actually employed by clinicians for managing a clinical problem, such as a disease or condition. This workflow data includes one or more of what care steps were performed, when they were performed, who performed them, what the result of performing them was, and the like. The workflow data is suitably stored in a workflow database **170.** However, it is contemplated that the workflow data is stored in other components of the medical institution. In certain embodiments, the workflow data is collected from the sourcing clinical device(s) and/or other components of the medical institution, such as the patient information system **106.** The workflow data is suitably collected electronically, but other approaches to collecting the workflow data are contemplated. For example, in certain embodiments, the workflow data is collected manually from, for example, a written form, and entered by a user into an electronic form for the CDS/WM system **110.** It is also possible that in certain embodiments, the function of the data collection engine is subsumed by the guideline execution engine **172** and workflow data stored as part of CIG instances.

In normal operation, the state of the guideline execution engine **172,** when applying a given CIG to a given patient, reflects the state of care for that patient; this state is represented by the instance of that CIG for that patient. Upon receiving new patient data, the guideline execution engine **172** applies that data to the CIG's logic, updates its status accordingly, and provides any resulting recommendations to the CDS/WM system **110.** In case the guideline execution engine **172** is involved partway through patient care however, the initially 'blank' state of the CIG engine (viz. the CIG instance) does not properly reflect the patient's actual state of care. In that case, the guideline execution engine **172** has to explicitly synchronize its state with the state of care. This is done by feeding the engine, and thereby the CIG, with historic patient data available from, for example, patient information system **106.** Crucially, this data must be applied in chronological order. Thus, the guideline execution engine **172** executes a synchronization procedure which loads the specified CIGs, associates it with the specified patient, and initiates the guideline execution engine **172** in 'Sync' mode. The 'Sync' mode indicating that the CIG is started partway patient care and instructing the guideline execution engine **172** to synchronize the CIG instance state with the current state of patient care. To accomplish this, the guideline execution engine **172** retrieves all relevant data for the current patient from the patient information system **106** and executes the CIG(s) to each datum in chronological order. Specifically, the guideline execution engine **172** processes patient data chronologically, i.e., in the temporal order that it was created.. This is crucial, because supplying data in different temporal orders can result in the CIG engine ending up in different states, in which case the CIG instance would not properly represent the patient's state of care. The guideline execution engine **172** further provides an overview of all recommendations generated during the synchronization process, including those which are presently relevant and those which were relevant earlier in the treatment.

In one embodiment, the guideline execution engine **172** utilizes timers, for example, to generate an alert when a certain amount of time has elapsed after a given event. Because the guideline execution engine **172** is not operating in real-time mode when synchronization is in progress, actual timers cannot be used. Instead, the guideline execution engine **172** inserts virtual timer events in the stream of patient data at the appropriate chronological locations. The actions associated with these events are stamped with the time at which the timer would have expired had the CIG been executed at the start of patient care. In case the guideline execution engine **172** involves timers that have not yet expired when synchronization completes, the guideline execution engine **172** instantiates and activates these timers, and sets their elapsed time to the time that would have elapsed had the CIG been executed at the start of patient care. Any recommendations generated in the synchronization process are appropriately stamped with predated times and collected in chronological order. Any of the collected recommendations that can be inferred (from patient data) to have been completed are marked as such. For example, a recommendation "Perform head CT scan" gets marked completed when the workflow data shows that the proposed CT scan has been done. The list of completed recommendations can be used for quality control purposes, e.g., to assess guideline adherence. The list of recommendations that have not been completed is provided to the user, typically for review by care providers. The synchronization process terminates when all historic patient data has been processed by the guideline execution engine **172.** The resulting state of the guideline execution engine **172,** and hence the associated CIG instance, represents the current state of patient care, i.e., it properly indicates the patient's position in the CIG. The guideline execution engine **172** now switches to real-time mode, responding to new patient data and commands as they arrive.

It is also contemplated that when CIG execution encounters a decision point, the guideline execution engine **172** utilizes the documented decision if it is available from the patient data, and otherwise infers what decision was made from subsequent patient data. If neither works, the guideline execution engine **172** requests a resolution from the environment (which typically involves prompting input from a care provider). To account for missing optional patient data, the guideline execution engine **172** may offer a provision to allow guideline steps to be skipped or partially completed. To account for missing mandatory patient data, the guideline execution engine **172** requests the data in question from the environment (which typically involves prompting input from a care provider). In case the care provider cannot provide the data that is needed, he or she can instruct the guideline execution engine **172** how to proceed (for which the engine may provide the available options). Missing data items could be ranked by a variable importance measure or a procedure like PCA (Principal Components Analysis) to determine the fewest/most relevant questions to ask the users/clinicians. It is also contemplated that a priori segmentation of the CIG could be performed to determine what patient data can be inferred and what data needs to be provided by a clinician. For example, in lung cancer treatment it is difficult to infer the treatment phase and such patient data needs to be provided by a clinician. Once the treatment phase is known, the location in that phase of the CIG can be inferred from patient data, when available.

Note that the synchronization procedure provides a more comprehensive solution than manually selecting an entry point, in the sense that it results in a state of the guideline execution engine **172** that approximates or is identical to the state that would have been obtained if the engine had executed the CIGs from the start of care, including the generation of any recommendations that would have been generated. The synchronization procedure does not require the CIG to have entry points other than the main starting point. It is also contemplated that the guideline execution engine **172** be modified to be applicable not only when it is first invoked partway patient care, but also when the guideline execution engine **172** loads a prior state that had been persisted for some time. In the latter case, the guideline execution engine **172** starts in the state that was persisted and subsequently applies any patient data that has become available since the time the state was persisted.

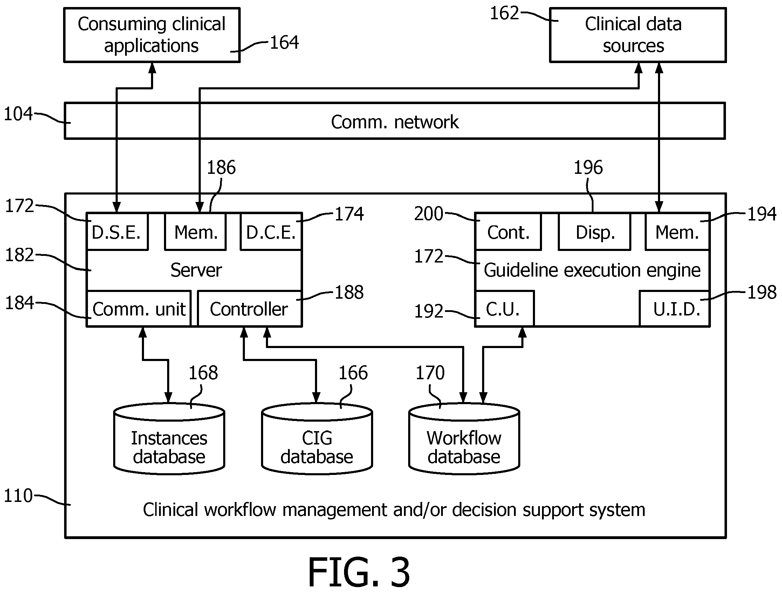

With reference to FIGURES 3, a structural view of the CDS/WM system **110** is provided. A server **182** of the CDS/WM system **110** suitably includes the guideline execution engine **172** and the data collection engine **174.** In certain embodiments, each of the guideline execution engine **172** and the data collection engine **174** is embodied by a non-transient computer readable medium having computer executable instructions for performing the tasks associated with the guideline execution engine **172** and/or the data collection engine **174.** A communications unit **184** of the server **182** facilitates communication between the server **182** and external devices, such as the clinical device(s) **102.** The communications unit **184** further facilitates communication with the databases **166, 168, 170** of the CDS/WM system **110.** A memory **186** of the server **182** stores executable instructions for performing one of more of the functions associated with the server **182.** In certain embodiments, these instructions include instructions for performing the tasks associated with the guideline execution engine **172** and/or the data collection engine **174.** A controller **188** of the server **182** executes instructions of the memory **186,** the guideline execution engine **172,** or the data collection engine **174.**

The CDS/WM system **110** suitably includes the guideline execution engine **172.** In certain embodiments, the guideline execution engine **172** is embodied by a non-transient computer readable medium having computer executable instructions for performing the tasks associated with the guideline execution engine **172.** A communications unit **192** of the guideline execution engine **172** facilitates communication between the guideline execution engine **172** and external devices, such as the clinical device(s) **102.** The communications unit **192** further facilitates communication with the databases **166, 168, 170** of the CDS/WM system **110.** A memory **194** of the guideline execution engine **172** stores executable instructions for performing one of more of the functions associated with the guideline execution engine **172.** In certain embodiments, these instructions include instructions for performing the tasks associated with the data collection engine **174.** A display **196** of the guideline execution engine **172** allows the CDS/WM system **110** to display a user interface allowing a user, such as a knowledge engineer, to interact with the guideline execution engine **172.** A user input device **198** of the guideline execution engine **172** allows the user to interact with the user interface. A controller **200** of the guideline execution engine **172** executes instructions of the memory **194.** It is also contemplated the server **182** and guideline execution engine **172** are embodied as a single device.

Each of the databases described herein, such as the CIG database **166,** suitably include a computer database, where the computer database is embodied by a single computer, distributed across a plurality of computers, or the like. Further, each of the databases suitably stores data in a structured manner facilitating recall and access to such data. Further, as used herein, a memory includes one or more of a non-transient computer readable medium; a magnetic disk or other magnetic storage medium; an optical disk or other optical storage medium; a random access memory (RAM), read-only memory (ROM), or other electronic memory device or chip or set of operatively interconnected chips; an Internet server from which the stored instructions may be retrieved via the Internet or a local area network; or so forth. Further, as used herein, a controller includes one or more of a microprocessor, a microcontroller, a graphic processing unit (GPU), an application-specific integrated circuit (ASIC), a field-programmable gate array (FPGA), and the like; a communications network includes one or more of the Internet, a local area network, a wide area network, a wireless network, a wired network, a cellular network, a data bus, such as USB and I2C, and the like; a user input device includes one or more of a mouse, a keyboard, a touch screen display, one or more buttons, one or more switches, one or more toggles, and the like; and a display includes one or more of a LCD display, an LED display, a plasma display, a projection display, a touch screen display, and the like.

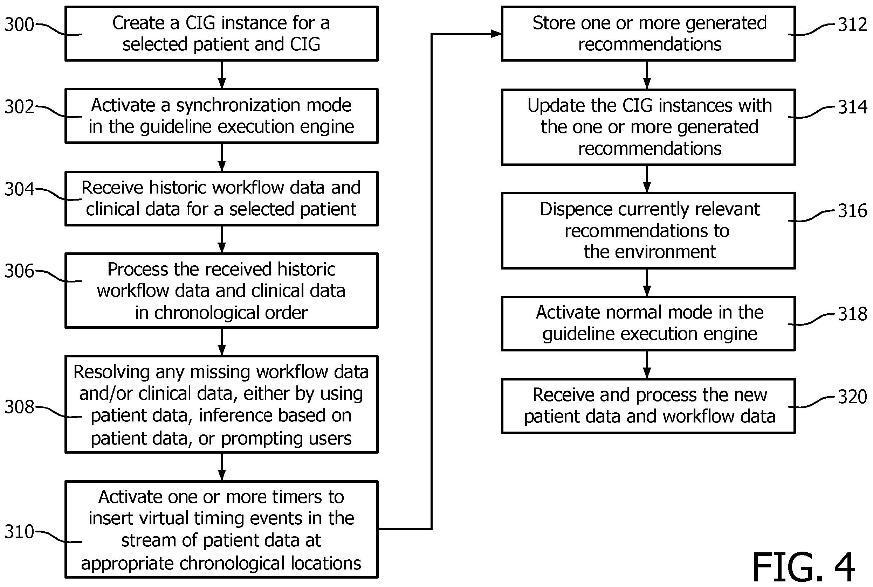

FIGURE 4 illustrates the synchronization procedure of the CDS/WM system. In a step **300,** a CIG instance is created for a selected patient and CIG. In a step **302,** synchronization mode in the guideline execution engine is activated. In a step **304,** historic workflow data and clinical data is received for a selected patient. The workflow data includes a plurality of care steps for each of the patients and the clinical data includes clinical measurements and observations collected from each of the patients. In a step **306,** the received historic workflow data and clinical data is processed in chronological order. This includes step **308,** in which any missing workflow data and/or clinical data are resolved, either by using clinical data, inference based on clinical data, or prompting users, as described above; and step **310,** in which one or more timers are activated to insert virtual timing events in the stream of clinical data at appropriate chronological locations. Step **306** may generate one or more recommendations, which are stored in step **312.** In a step **314,** the CIG instances, updated by step **306,** are stored in a database. In a step **316,** currently relevant recommendations are dispensed to the environment. In a step **318,** normal mode in the guideline execution engine is activated. In a step **320,** new clinical data and workflow data is received and processed.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims.

## Claims

1. A method of synchronizing a state of a computer interpretable guideline engine with a state of patient care, the method comprising:
receiving historic patient data for one or more patients, wherein patient data includes workflow data and clinical data, wherein the workflow data includes information on a plurality of care steps for each of the patients and the clinical data includes clinical measurements and observations collected from each of the patients;
resolving missing patient data for each of the patients utilizing historic patient data, inference based on historic patient data, or prompting users;
updating the engine state utilizing the resolved patient data;
receiving current patient data for one or more patients; and
updating the engine state utilizing the current patient data,
**characterized in that** missing data is ranked by Principal Components Analysis to determine relevant questions to ask the users,
wherein a priori segmentation of the computer interpretable guideline is performed to determine which patient data can be inferred and which patient data needs to be provided by the users.

2. The method according to claim 1, wherein each of the care steps and the logic in the computer interpretable guideline are based on established clinical guidelines and /or protocols.

3. The method according to either one of claims 1 and 2, further including:
processing the patient data in chronological order.

4. The method according to any one of claims 1-3, wherein the step of synchronizing the state of the computer interpretable guideline engine with the state of patient care further includes using historic patient data:
inferring part of the state from the historic patient data.

5. The method according to any one of claims 1-4, wherein the step of synchronizing the state of the computer interpretable guideline engine with the state of patient care further includes:
querying a clinician if information on one or more care steps or one or more measurements or observations, cannot be inferred from the historic patient data.

6. The method according to any one of claims 1-5, wherein the step of synchronizing state of the computer interpretable guideline engine with the state of patient care further includes:
inserting one or more virtual timer events the historic patient data as a result of applying historic patient data to the computer interpretable guideline's logic.

7. The method according to claim 1-6, further including:
activating one or more real-time timers as a result of applying historic patient data to the computer interpretable guideline's logic.

8. A computer readable medium containing software which when loaded into processor programs the processor to perform the method according to any one of claims 1-7.

9. A clinical decision support or workflow management system comprising:
a patient information system which stores historical workflow data and patient data; and
one or more processors programmed to perform the method according to any one of claims 1-7.

## Patentansprüche

1. Verfahren zum Synchronisieren eines Zustands einer Engine für computerinterpretierbare Richtlinien mit einem Patientenversorgungszustand, wobei das Verfahren umfasst:
Empfangen von historischen Patientendaten für einen oder mehrere Patienten, wobei die Patientendaten Workflow-Daten und klinische Daten einschließen, wobei die Workflow-Daten Informationen über eine Vielzahl von Versorgungsschritten für jeden der Patienten einschließen und die klinischen Daten klinische Messungen und Beobachtungen einschließen, die von jedem der Patienten erfasst wurden;
Klären fehlender Patientendaten für jeden der Patienten unter Verwendung von historischen Patientendaten, Schlüssen, die aus den historischen Patientendaten gezogen werden, oder durch Befragung von Benutzern;
Aktualisieren des Engine-Zustands unter Verwendung der geklärten Patientendaten;
Empfangen von aktuellen Patientendaten für einen oder mehrere Patienten; und
Aktualisieren des Engine-Zustands unter Verwendung der aktuellen Patientendaten,
**dadurch gekennzeichnet, dass** fehlende Daten durch Hauptkomponentenanalyse ihrer Bedeutung nach eingestuft werden, um relevante Fragen zu bestimmen, die Benutzern zu stellen sind,
wobei eine Vorabsegmentierung der computerinterpretierbaren Richtlinie durchgeführt wird, um zu bestimmen, welche Patientendaten erschlossen werden können und welche Patientendaten von den Benutzern bereitgestellt werden müssen.

2. Verfahren nach Anspruch 1, wobei jeder von den Versorgungsschritten und die Logik in der computerinterpretierbaren Richtlinie auf etablierten klinischen Richtlinien und/oder Protokollen basieren.

3. Verfahren nach einem der Ansprüche 1 bis 2, ferner einschließend:
Verarbeiten der Patientendaten in chronologischer Ordnung.

4. Verfahren nach einem der Ansprüche 1-3, wobei der Schritt des Synchronisierens des Zustands der Engine für computerinterpretierbare Richtlinien mit dem Patientenversorgungszustand ferner die Verwendung historischer Patientendaten einschließt:
Erschließung eines Teils des Zustands aus den historischen Patientendaten.

5. Verfahren nach einem der Ansprüche 1-4, wobei der Schritt des Synchronisierens des Zustands der Engine für computerinterpretierbare Richtlinien mit dem Patientenversorgungszustand ferner einschließt:
Befragen eines Arztes, falls Informationen über einen oder mehrere Versorgungsschritt oder eine oder mehrere Messungen oder Beobachtungen nicht aus den historischen Patientendaten geschlossen werden können.

6. Verfahren nach einem der Ansprüche 1-5, wobei der Schritt des Synchronisierens des Zustands der Engine für computerinterpretierbare Richtlinien mit dem Patientenversorgungszustand ferner einschließt. Einfügen eines oder mehrerer virtueller Zeitgeberereignisse der historischen Patientendaten als Ergebnis der Anwendung von historischen Patientendaten auf die Logik der computerinterpretierbaren Richtlinie.

7. Verfahren nach Anspruch 1-6, ferner einschließend:
Aktivieren eines oder mehrerer virtueller Zeitgeber als Ergebnis der Anwendung von historischen Patientendaten auf die Logik der computerinterpretierbaren Richtlinie.

8. Computerlesbares Medium, das Software enthält, die, wenn sie in Prozessorprogramme geladen wird, den Prozessor dafür programmiert, das Verfahren nach einem der Ansprüche 1-7 durchzuführen.

9. System zur Unterstützung klinischer Entscheidungen oder zur Verwaltung von klinischen Workflows, umfassend:
ein Patienteninformationssystem, das historische Workflow-Daten und Patientendaten speichert; und
einen oder mehrere Prozessoren, die dafür programmiert sind, das Verfahren nach einem der Ansprüche 1-7 durchzuführen.

## Revendications

1. Procédé de synchronisation d'un état d'un moteur de lignes de guidage pouvant être interprétées par ordinateur avec un état de soins aux patients, ledit procédé comprenant :
la réception des données patient historiques pour au moins un patient, dans lequel les données patient comprennent des données de flux de travail et des données cliniques, dans lequel les données de flux de travail comprennent des informations sur une pluralité d'étapes de soins pour chacun des patients et les données cliniques comprennent des mesures cliniques et des observations collectées auprès de chacun des patients ;
la résolution des données patient manquantes pour chacun des patients à l'aide des données patient historiques, d'une inférence basée sur des données patient historiques ou des utilisateurs incitatifs ;
la mise à jour de l'état du moteur à l'aide les données patient résolues ;
la réception des données actuelles des patients pour au moins un patient ; et la mise à jour de l'état du moteur à l'aide les données patient actuelles,
**caractérisé en ce que** les données manquantes sont classées par analyse en composantes principales pour déterminer les questions pertinentes à poser aux utilisateurs,
dans lequel une segmentation a priori de la ligne de guidage pouvant être interprétée par ordinateur est réalisée pour déterminer quelles données patient peuvent être déduites et quelles données patient doivent être fournies par les utilisateurs.

2. Procédé selon la revendication 1, dans lequel chacune des étapes de soins et la logique de la ligne de guidage pouvant être interprétée par ordinateur sont basées sur des lignes de guidages cliniques et/ou des protocoles établis.

3. Procédé selon l'une quelconque des revendications 1 et 2, comprenant en outre :
le traitement des données patient dans l'ordre chronologique.

4. Procédé selon l'une quelconque des revendications 1 à 3, dans lequel l'étape de synchronisation de l'état du moteur de lignes de guidage pouvant être interprétées par ordinateur avec l'état de soins aux patients comprend en outre l'utilisation de données patient historiques :
la déduction d'une partie de l'état à partir des données patient historiques.

5. Procédé selon l'une quelconque des revendications 1 à 4, dans lequel l'étape de synchronisation de l'état du moteur de lignes de guidage pouvant être interprétées par ordinateur avec l'état de soins aux patients comprend en outre :
l'interrogation d'un clinicien si des informations sur au moins une étape de soins ou au moins une mesure ou observation ne peuvent pas être déduites des données patient historiques.

6. Procédé selon l'une quelconque des revendications 1 à 5, dans lequel l'étape de synchronisation de l'état du moteur de lignes de guidage pouvant être interprétées par ordinateur avec l'état de soins aux patients comprend en outre :
l'insertion d'au moins un événement de minuterie virtuelle dans les données patient historiques suite à l'application des données patient historiques à la logique de la ligne directrice pouvant être interprétée par ordinateur.

7. Procédé selon les revendications 1 à 6, comprenant en outre :
l'activation d'au moins une minuterie en temps réel suite à l'application des données patient historiques à la logique de la ligne de guidage pouvant être interprétée par ordinateur.

8. Support lisible par ordinateur contenant un logiciel, lequel, lorsqu'il est chargé dans le processeur, programme le processeur pour mettre en œuvre le procédé selon l'une quelconque des revendications 1 à 7.

9. Système d'aide à la décision clinique ou de gestion de flux de travail comprenant :
un système d'information patient, lequel mémorise les données de flux de travail historiques et les données patient ; et
au moins un processeur programmé pour mettre en œuvre le procédé selon l'une quelconque des revendications 1 à 7.
